# EUROPÄISCHE PATENTANMELDUNG

(11) **EP 1 522 855 A2**
(43) Veröffentlichungstag der Anmeldung: **13.04.2005**
(21) Anmeldenummer: 04023017.9
(22) Anmeldetag: 28.09.2004
(51) Int. Cl.: G01N 33/60, C07K 14/435, C07K 14/705, C07C 233/65

(54) **Verfahren zum Identifizieren von pestiziden Wirkstoffen**

(30) Priorität: 10.10.2003 DE 10347089; 04.12.2003 DE 10356553
(71) Anmelder: Bayer CropScience AG, 40789 Monheim (DE)
(72) Erfinder: Schulte, Thomas, Dr., 51061 Köln (DE); Ebbinghaus-Kintscher, Ulrich, Dr., 44287 Dortmund (DE); Lümmen, Peter, Dr., 65510 Idstein (DE); Fischer, Rüdiger, Dr., 50259 Pulheim (DE); Funke, Christian, Dr., 42799 Leichlingen (DE)

(57) **Zusammenfassung**

Die vorliegende Erfindung betrifft ein Verfahren zum Identifizieren von pestiziden Wirkstoffen. Gegenstand der vorliegenden Erfindung ist insbesondere ein Bindungstest mit markierten Phthalsäurediamiden an Membranpräparationen und die Verdrängung dieser markierten Phthalsäurediamide durch unmarkierte Testsubstanzen.

## Beschreibung

Die vorliegende Erfindung betrifft ein Verfahren zum Identifizieren von pestiziden Wirkstoffen.

Verbindungen aus der Klasse der Phthalsäurediamide (vgl. EP-A-0 919 542, EP-A-1 006 107, WO 01/00575, WO 01/00599, WO 01/46124, JP 2001-33 555 9, WO 01/02354, WO 01/21576, WO 02/088074, WO 02/088075, WO 02/094765, WO 02/094766, WO 02/062807) sind hochpotente Pestizide. Der Wirkmechanismus dieser Verbindungen ist noch nicht öffentlich bekannt und stellt ein neues Wirkprinzip dar. Ein Hochdurchsatz-Screeningverfahren zum Identifizieren von Wirkstoffen, die dem gleichen Mechanismus unterliegen, ist daher von hohem Wert.

Aufgabe der vorliegenden Erfindung ist daher die Bereitstellung eines Verfahrens, womit pestizide Wirkstoffe identifiziert werden können, die durch Beeinflussung des gleichen Angriffpunkts (Targets) wie derjenige der Phthalsäurediamide ihre Wirkung entfalten.

Die Aufgabe wird gelöst durch Bereitstellung eines Verfahrens, welches dadurch gekennzeichnet ist, dass man die Verdrängung einer i) markierten Verbindung gemäß der nachstehenden allgemeinen Formel (I) oder ii) einer markierten Verbindung, die an die gleiche Bindungsstelle bindet wie eine Verbindung gemäß der allgemeinen Formel (I) an Membranpräparationen oder Zelllinien, die das molekulare Target der Verbindungen gemäß der allgemeinen Formel (I) enthalten, misst: 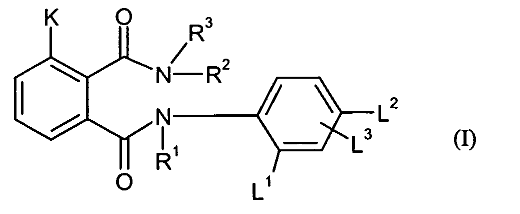 in welcher
- K: für Halogen, Cyano, Alkyl, Halogenalkyl, Alkoxy oder Halogenalkoxy steht,
- R¹, R², R³: jeweils unabhängig voneinander für Wasserstoff, Cyano, für gegebenenfalls durch Halogen substituiertes C₃-C₈-Cycloalkyl oder für eine Gruppe der Formel M¹-Qₖ stehen, in welcher
M¹ für gegebenenfalls substituiertes Alkylen, Alkenylen oder Alkinylen steht,
Q für Wasserstoff, Halogen, Cyano, Nitro, Halogenalkyl, jeweils gegebenenfalls substituiertes C₃-C₈-Cycloalkyl, Alkylcarbonyl oder Alkoxycarbonyl, jeweils gegebenenfalls substituiertes Phenyl, Hetaryl oder für eine Gruppe T-R⁴ steht, in welcher
T für -O-, -S(O)ₘ- oder 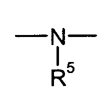 steht,
R⁴ für Wasserstoff, jeweils gegebenenfalls subsitutiertes Alkyl, Alkenyl, Alkinyl, Cycloalkyl, Cycloalkyl-alkyl, Alkoxyalkyl, Alkylcarbonyl, Alkoxycarbonyl, Phenyl, Phenylalkyl, Phenylalkoxy, Hetaryl, Hetarylalkyl steht,
R⁵ für Wasserstoff, für jeweils gegebenenfalls substituiertes Alkylcarbonyl, Alkoxycarbonyl, Phenylcarbonyl oder Phenylalkoxycarbonyl steht,
k für die Zahlen 1 bis 4 steht,
m für die Zahlen 0 bis 2 steht, oder
- R¹ und R²: gemeinsam einen gegebenenfalls substituierten vier- bis siebengliedrigen Ring bilden, der gegebenenfalls durch Heteroatome unterbrochen sein kann,
- L¹ und L³: unabhängig voneinander für Wasserstoff, Halogen, Cyano oder jeweils gegebenenfalls substituiertes Alkyl, Alkoxy, Alk-S(O)ₘ- , Phenyl, Phenoxy oder Hetaryloxy stehen,
- _{L}2: für Wasserstoff, Halogen, Cyano, jeweils gegebenenfalls substituiertes Alkyl, Alkenyl, Alkinyl, Halogenalkyl, Cycloalkyl, Phenyl, Hetaryl oder für die Gruppe M²-R⁶ steht, in welcher
M² für -O- oder -S(O)ₘ- steht,
R⁶ für jeweils gegebenenfalls substituiertes Alkyl, Alkenyl, Alkinyl, Cycloalkyl, Phenyl oder Hetaryl steht, oder
- L¹ und L³ oder L¹ und L²: gegebenenfalls gemeinsam einen gegebenenfalls substituierten fünf- bis sechsgliedrigen Ring bilden, der gegebenenfalls durch Heteroatome unterbrochen sein kann.

Bevorzugte Substituenten bzw. Bereiche der in der allgemeinen Formel (I) aufgeführten Reste werden im Folgenden erläutert.
- K: steht bevorzugt für Fluor, Chlor, Brom, Jod, Cyano, C₁-C₆-Alkyl, C₁-C₆-Halogenalkyl, C₁-C₆-Alkoxy oder C₁-C₆-Halogenalkoxy.
- R¹, R², R³: stehen jeweils unabhängig voneinander bevorzugt für Wasserstoff, Cyano, für gegebenenfalls durch Halogen substituiertes C₃-C₆-Cycloalkyl oder für eine Gruppe der Formel M¹-Qₖ, in welcher
M¹ für C₁-C₈-Alkylen, C₃-C₆-Alkenylen oder C₃-C₆-Alkinylen steht,
Q für Wasserstoff, Halogen, Cyano, Nitro, Halogenalkyl oder für gegebenenfalls durch Fluor, Chlor, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen substituiertes C₁-C₆-Alkylcarbonyl oder C₁-C₆-Alkoxycarbonyl oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl oder Hetaryl mit 5 bis 6 Ringatomen oder für eine Gruppe T-R⁴ steht, in welcher
T für -O-, -S(O)ₘ- oder 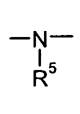 steht,
R⁴ für Wasserstoff, für jeweils gegebenenfalls durch Fluor und/oder Chlor substituiertes C₁-C₈-Alkyl, C₃-C₈-Alkenyl, C₃-C₈-Alkinyl, C₃-C₈-Cycloalkyl, C₃-C₈-Cycloalkyl-C₁-C₂-alkyl, C₁-C₆-Alkylcarbonyl, C₁-C₆-Alkoxycarbonyl, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, C₁-C₄-Phenylalkyl, C₁-C₄-Phenylalkyloxy, Hetaryl oder Hetarylalkyl steht, wobei Hetaryl 5 bis 6 Ringatome umfasst und insbesondere für Furanyl, Pyridyl, Imidazolyl, Triazolyl, Pyrazolyl, Pyrimidyl, Thiazolyl oder Thienyl steht,
R⁵ für Wasserstoff, für jeweils gegebenenfalls durch Fluor und/oder Chlor substituiertes C₁-C₆-Alkylcarbonyl, C₁-C₆-Alkoxycarbonyl, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenylcarbonyl oder Phenyl-C₁-C₄-alkyloxycarbonyl steht,
k für die Zahlen 1 bis 3 steht, und
m für die Zahlen 0 bis 2 steht.
- R¹ und R²: können auch einen fünf- bis sechsgliedrigen Ring bilden, der gegebenenfalls durch ein Sauerstoff- oder Schwefelatom unterbrochen sein kann.
- L¹ und L³: stehen unabhängig voneinander bevorzugt für Wasserstoff, Fluor, Chlor, Brom, Jod, Cyano, C₁-C₆-Alkyl, C₁-C₄-Halogenalkyl, C₁-C₆-Alkoxy, C₁-C₄-Halogenalkoxy, C₁-C₄-Alkyl-S(O)ₘ-, C₁-C₄-Haloalkyl-S(O)ₘ-, für jeweils gegebenenfalls einfach bis dreifach durch Fluor, Chlor, Brom, C₁-C₆-Alkyl, C₁-C₆-Alkoxy, C₁-C₄-Halogenalkyl, C₁-C₄-Halogenalkoxy, Cyano oder Nitro substituiertes Phenyl, Phenoxy, Pyridyloxy, Thiazolyloxy oder Pyrimidyloxy.
- L²: steht bevorzugt für Wasserstoff, Fluor, Chlor, Brom, Jod, Cyano, für jeweils gegebenenfalls durch Fluor und/oder Chlor substituiertes C₁-C₁₀-Alkyl, C₂-C₁₀-Alkenyl, C₂-C₆-Alkinyl, für gegebenenfalls durch Fluor oder Chlor substituiertes C₃-C₆-Cycloalkyl, für jeweils gegebenenfalls einfach bis dreifach durch Fluor, Chlor, Brom, C₁-C₆-Alkyl, C₁-C₆-Alkoxy, C₁-C₄-Halogenalkyl, C₁-C₄-Halogenalkoxy, Cyano oder Nitro substituiertes Phenyl, Pyridyl, Thienyl, Pyrimidyl oder Thiazolyl, oder für eine Gruppe M²-R⁶, in welcher
M² für -O- oder -S(O)ₘ- steht, und
R⁶ für jeweils gegebenenfalls durch Fluor und/oder Chlor substituiertes C₁-C₈-Alkyl, C₂-C₈-Alkenyl, C₃-C₆-Alkinyl oder C₃-C₆-Cycloalkyl, für jeweils gegebenenfalls einfach bis dreifach durch Fluor, Chlor, Brom, C₁-C₆-Alkyl, C₁-C₆-Alkoxy, C₁-C₄-Halogenalkyl, C₁-C₄-Halogenalkoxy, Cyano oder Nitro substituiertes Phenyl, Pyridyl, Pyrimidyl oder Thiazolyl steht.
- L¹ und L³ oder L² und L³: können auch gemeinsam jeweils einen gegebenenfalls durch Fluor und/oder C₁-C₂-Alkyl substituierten fünf- bis sechsgliedrigen Ring bilden, der gegebenenfalls durch ein oder zwei Sauerstoffatome unterbrochen sein kann.
- K: steht besonders bevorzugt für Chlor, Brom oder Jod.
- R¹, R², R³: stehen jeweils unabhängig voneinander besonders bevorzugt für Wasserstoff oder für eine Gruppe der Formel M¹-Qₖ, in welcher
M¹ für C₁-C₈-Alkylen, C₃-C₆-Alkenylen oder C₃-C₆-Alkinylen steht,
Q für Wasserstoff, Fluor, Chlor, Cyano, Trifluormethyl, C₃-C₆-Cycloalkyl oder für eine Gruppe T-R⁴ steht, in welcher
T für -O- oder -S(O)ₘ- steht,
R⁴ für Wasserstoff, jeweils gegebenenfalls einfach bis dreifach durch Fluor und/oder Chlor substituiertes C₁-C₆-Alkyl, C₃-C₆-Alkenyl, C₃-C₆-Alkinyl oder C₃-C₆-Cycloalkyl steht,
k für die Zahlen 1 bis 3 steht, und
m für die Zahlen 0 bis 2 steht.
- L¹ und L³: stehen unabhängig voneinander besonders bevorzugt für Wasserstoff, Fluor, Chlor, Brom, Jod, Cyano, C₁-C₄-Alkyl, C₁-C₂-Halogenalkyl, C₁-C₄-Alkoxy, C₁-C₂-Halogenalkoxy, für jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl oder Phenoxy.
- L²: steht besonders bevorzugt für Wasserstoff, Fluor, Chlor, Brom, Jod, Cyano, für jeweils gegebenenfalls einfach bis dreizehnfach durch Fluor und/oder Chlor substituiertes C₁-C₆-Alkyl, C₂-C₆-Alkenyl, C₂-C₆-Alkinyl, C₃-C₆-Cycloalkyl oder für eine Gruppe M²-R⁶, in welcher
M² für -O- oder -S(O)ₘ- steht, und
R⁶ für jeweils gegebenenfalls einfach bis dreizehnfach durch Fluor und/oder Chlor substituiertes C₁-C₆-Alkyl, C₂-C₆-Alkenyl, C₂-C₆-Alkinyl oder C₃-C₆-Cycloalkyl, für jeweils gegebenenfalls einfach bis zweifach durch Fluor, Chlor, Brom, C₁-C₄-Alkyl, C₁-C₄-Alkoxy, Trifluormethyl, Difluormethoxy, Trifluormethoxy, Cyano oder Nitro substituiertes Phenyl oder Pyridyl steht.

Ganz besonders bevorzugt handelt es sich bei der Verbindung gemäß der allgemeinen Formel (I) um die folgende Verbindung: 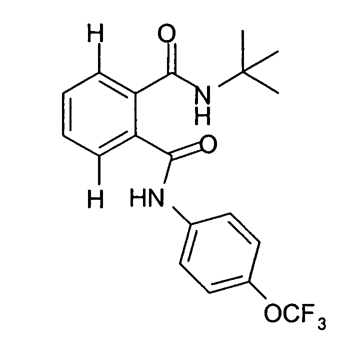 Bei den Verbindungen der allgemeinen Formel I handelt es sich um bekannte Verbindungen, die in den folgenden Publikationen beschrieben sind oder von diesen umfasst werden (vgl. EP-A-0 919 542, EP-A-1 006 107, WO 01/00575, WO 01/00599, WO 01/46124, JP 2001-335559, WO 01/02354, WO 01/21576, WO 02/088074, WO 02/088075, WO 02/094765, WO 02/094766, WO 02/062807). Sie können nach den darin beschriebenen Verfahren hergestellt werden.

Gegenstand der vorliegenden Erfindung sind also Bindungstests mit markierten Phthalsäurediamiden an Membranpräparationen von geeigneten Organismen oder Zelllinien, die das molekulare Target der Phthalsäurediamide enthalten, und die Verdrängung dieser markierten Phthalsäurediamide durch unmarkierte Testsubstanzen. Bei solchen Test- oder Kandidaten-Verbindungen kann es sich prinzipiell um jede beliebige Verbindung handeln, wobei diese zu den unterschiedlichsten Verbindungstypen zählen können. Der Fachmann kennt eine Vielzahl von Quellen, die für das erfindungsgemäße Verfahren geeignete Verbindungen enthalten. Prinzipiell eignen sich z.B. alle denkbaren Substanz-Bibliotheken; bevorzugt sind Bibliotheken mit niedermolekularen Verbindungen, insbesondere kleinen organisch-chemischen Verbindungen. Eine mit hoher Spezifität an das Target bindende Phthalsäurediamid-Verbindung kann gegebenenfalls durch die Testsubstanz aus der Bindungsstelle verdrängt werden. Unter Markierung ist allgemein eine den Nachweis erleichternde Modifikation an dem zu verdrängenden Molekül zu verstehen. Beispiele sind radioaktive, fluoreszierende oder lumineszierende Markierungen.

Die vorliegende Erfindung basiert auf der Erkenntnis, dass das molekulare Target der Phthalsäurediamide membranständig und durch einen Bindungs-/Verdrängungstest adressierbar ist.

Im Folgenden wird der theoretische Hintergrund des erfindungsgemäßen Verfahrens beschrieben. Dabei wird für eine Verbindung der allgemeinen Formel (I) auch die Bezeichnung "Ligand" verwendet:
Die einfache Okkupationstheorie beschreibt die reversible Bindung eines Liganden an einen Rezeptor in der Form eines Geschwindigkeitsgesetzes zweiter Ordnung. In Bindungsexperimenten wird allgemein die Ligandkonzentration im großen Überschuss relativ zur Rezeptorkonzentration eingestellt, so dass die Konzentration des freien Liganden in erster Näherung als konstant angesehen werden kann. Die Kinetik der Bindung ist somit nur noch von der Rezeptorkonzentration abhängig (Geschwindigkeitsgesetz pseudo-erster Ordnung).

Nach dem Massenwirkungsgesetz wird die Lage des Bindungsgleichgewichts durch die Dissoziationskonstante (Kehrwert der Assoziationskonstante) K_{d} = Kₐ⁻¹ = k_{off}/kₒₙ= [R]·[L]/[RL] beschrieben; k_{off} ist die Dissoziationsrate, kₒₙ ist die Assoziationsrate, [R] ist die Konzentration der freien Rezeptoren, [L] ist die Konzentration des nicht gebundenen Liganden, [RL] ist die Konzentration des Rezeptor-Ligand-Kompexes. Sie steht zur freien Energie der Bindung in proportionaler Beziehung: ΔG₀ = R·T·ln K_{d}; ΔG₀ ist die freie ("Gibbs"-)Energie der Bindung, R ist die allgemeine Gaskonstante: 8.315 J mol⁻¹ K⁻¹, T ist die absolute Temperatur (in Kelvin) [Repke, H., C. Liebmann; Membranrezeptoren und ihre Effektorsysteme; VCH (Weinheim), 1987]. Entsprechend bedeutet eine 10fach erniedrigte K_{d} einen Gewinn von -5.71 kJ mol⁻¹ K⁻¹ an freier Bindungsenergie.

In Sättigungs-Bindungsexperimenten wird die Konzentration des spezifisch gebundenen Liganden [RL] als Funktion der Ligandkonzentration [L] bei konstanter Rezeptorkonzentration bestimmt. Für die Bestimmung der Konzentraton ist der Ligand z.B. durch den Einbau eines Radioisotops oder durch Substitution mit einem Fluorophor markiert. Die Auftragung von [RL] gegen [L] ergibt eine typische Sättigungskurve. Für den Fall [L] = K_{d} gilt: [RL] =½ [Rₜ]; [Rₜ], die Gesamtkonzentration der Rezeptoren, ist die Summe aus der Konzentration der freien Rezeptoren [R] und der Konzentration der Rezeptor-Ligand-Komplexe [RL]. In Gegenwart sättigender Ligandkonzentrationen entspricht die Konzentration der Rezeptor-Ligand-Komplexe der Gesamtkonzentration der Rezeptoren, häufig auch als maximale Bindung (Bₘₐₓ) bezeichnet.

Aus der Anpassung der Daten an ein hyperbolisches Modell mit Hilfe geeigneter PC-Programme, z.B. EBDA/LIGAND (BioSoft) oder SigmaPlot (SPSS), werden die kinetischen Gleichgewichtsparameter K_{d} und Bₘₐₓ berechnet.

Kompetitionsexperimente beschreiben den Einfluss unmarkierter Testsubstanzen (bezeichnet als Inhibitor, I) auf die Bindung des markierten Liganden. Im einfachsten Fall konkurriert der Inhibitor mit dem markierten Liganden um die gemeinsame Bindungsstelle.

Exprimentell wird die Verminderung der spezifischen Bindung des markierten Liganden als Funktion der Inhibitorkonzentration bestimmt. Die Auftragung der spezifischen Bindung gegen den Logarithmus der Inhibitorkonzentration ergibt eine charakteristische sigmoide Kurve, deren Wendepunkt die Inhibitorkonzentration kennzeichnet, bei der 50 % der Ligandenbindung gehemmt wird (IC₅₀-Wert). Die Dissoziationskonstante des Inhibitors Kᵢ steht zur Ligandkonzentration [L] und zur Dissoziationskonstanten des markierten Liganden K_{d} in Beziehung: Kᵢ = IC₅₀/(1 + [L]/K_{d}) [Cheng, Y., W.H. Prusoff; Biochem. Pharmacol. 22, 3099-3108 (1973)].

Mit Hilfe von Kompetitions-Screenings, die prinzipiell automatisierbar und damit im Hochdurchsatzverfahren durchführbar sind, werden Rezeptorliganden mit neuen Strukturen und Eigenschaften identifiziert.

Das erfindungsgemäße Verfahren kann unter Anwendung der im Folgenden beschriebenen Methoden durchgeführt werden.

Radioaktive Markierung des Liganden (d.h. einer Verbindung der allgemeinen Formel (I)) und Szintillationsmessung:

Der Einbau von Radioisotopen ist nach wie vor die häufigste Markierungsmethode für Rezeptorliganden in affinitätsbasierten Hochdurchsatz-Screenings. Tritium (³H) und ¹²⁵I genügen der Anforderung an hohe spezifische Radioaktivität der Liganden (> 30 Ci mmol⁻¹), die auch in nanomolaren Testkonzentrationen ein ausreichend großes Signal-Rausch-Verhältnis erlaubt. Insbesondere die Tritiummarkierung verändert die biochemischen Eigenschaften der Liganden nicht und ist durch radiochemische Standardmethoden (Direkteinbau, Halogen-Tritium-Austausch, katalytische Hydrierung) relativ leicht und kostengünstig darstellbar. Ein Vorteil der Tritium- gegenüber der Iod-Markierung ist mit Blick auf die radiochemische Stabilität die lange Halbwertszeit (12,3 Jahre, zum Vergleich: Halbwertszeit von ¹²⁵I beträgt 60 Tage).

Allen konventionellen radioaktiven Bindungtests gemeinsam ist die Notwendigkeit zur Trennung von gebundenem und freiem Liganden. Dies kann durch Gleichgewichtsdialyse, Zentrifugation, Gelfiltration oder Filtration über geeignete Membranen erreicht werden [Keen, M. (ed.); Receptor Binding Techniques; Meth. Molecular Biology, Vol. 106, Humana Press, Totowa, NJ (1999)].

Von den genannten Verfahren ist beispielsweise die Filtrationsmethode sinnvoll miniaturisierbar (Mikrotiterplattenformat) und automatisierbar (Roboter).

Mit der Zielrichtung des Hochdurchsatz-Screenings umfangreicher Substanz-Bibliotheken wurden homogene Testverfahren entwickelt, bei denen auf die Trennung gebundener und freier Liganden verzichtet werden kann. Weit verbreitet ist das von Amersham Biosciences als *Scintillation Proximity Assay* (SPA) kommerzialisierte System. Die Rezeptoren, solubilisiert, membrangebunden, aus nativen Geweben präpariert oder rekombinant exprimiert, werden auf sphärischen Partikeln immobilisiert, die einen Festphasenszintillator enthalten. Bindet der Radioligand, erzeugen die emittierten ß-Partikel (Elektronen) ein Lichtsignal im Szintillator, das mit einem β-Counter gemessen wird [Nelson, N.; Anal.Biochem. 165, 287-293 (1987), Alouani, S.; Meth.Mol.Biol. 138, 135-141 (2000)].

Im Folgenden sind Beispiele für Rezeptoren erwähnt, bei denen die SPA-Methode für Forschungsfragestellungen oder Screening eingesetzt wurde: IP₃-Rezeptor [Patel, S., et al.; Br.J.Pharmacol. 115, Proc.Suppl. 35p. (1995)], 5-HTle-Rezeptor [Kahl, S.D., et al.; J.Biomol.Screening 2, 33-40 (1995)], Lingandenscreening mit FKBP-12 [Graziani, F., et al.; J.Biomol.Screening 4, 3-7 (1999)], α-adrenerge Rezeptoren [Gobel, J., et al.; J.Pharmacol.Toxicol.Meth. 42, 237-244 (1999)], GABA_{B}-Rezeptoren [Urwyler, S., et al.; Mol.Pharmacol. 60, 963-971 (2001)].

Nach einem ähnlichen Prinzip funktionieren die FlashPlates (Perkin Elmer/NEN), bei denen die Rezeptoren in Mikrotiterplaten-Kavitäten immobilisiert werden. Die von gebundenen Radioliganden emittierten ß-Teilchen regen den Festphasenszintillator in den Mikrotiterplatten an.

Von den verschiedenen fluoreszenzbasierenden Detektionsmethoden ist insbesondere die Fluoreszenzpolarisation (FP) für Bindungstests geeignet. Eigenfluoreszente bzw. mit einem Fluorophor markierte Liganden werden mit linear polarisiertem Licht angeregt. Das von den frei diffundierenden, angeregten Ligandenmolekülen emittierte Licht besitzt eine wesentlich geringere Anisotropie, da die Moleküle während der Lebensdauer des angeregten Zustands Rotationsbewegungen und Schwingungen ausführen. Die Rotation rezeptorgebundener Liganden ist demgegenüber stark eingeschränkt, so dass ihre Fluoreszenz einen wesentlich höheren Polarisationsgrad (Anisotropie) aufweist, der etwa demjenigen der anregenden Strahlung entspricht. Die Messung der Fluoreszenzpolarisation ermöglicht eine direkte Bestimmung der Konzentrationen des gebundenen bzw. des freien Liganden [Sundberg, S.S.; Curr.Opinion Biotechnol. 11, 47-53 (2000)].

In der Literatur sind FP-basierende Hochdurchsatz-Bindungstests für lösliche Rezeptoren, z.B. Steroidrezeptoren [Parker, G.J., et al.; J.Biomol.Screen. 5, 77-88 (2000)], und membrangebundene Rezeptoren, z. B. G-Protein-gekoppelte Rezeptoren [Allen, M., et al.; J.Biomol.Screen. 5, 63-70 (2000), Banks, P., et al.; J.Biomol.Screen. 5, 159-168 (2000)] beschrieben.

FP-Bindungstests zeichnen sich generell durch hohe Empfindlichkeit im sub-nanomolaren Konzentrationsbereich und ein gutes Signal-Rausch-Verhältnis aus. Allerdings können fluoreszierende Testsubstanzen mit dem Messsignal interferieren.

Im Folgenden wird beschrieben, auf welche Weise man das biologische Material, insbesondere die Membranpräparationen, für das erfindungsgemäße Verfahren erhalten kann.

Tierische Schädlinge, insbesondere agronomisch relevante Schadorganismen, wie z.B. Pflanzenoder Saatgut-schädigende Insekten (insbesondere der Ordnungen Homoptera, Lepidoptera und Coleoptera), Spinnentiere (insbesondere Acarina) und pflanzenparasitäre Nematoden, aber auch biochemisch bzw. molekularbiologisch gut untersuchte Modellinsekten wie Taufliegen (*Drosophila melanogaster*), Stubenfliegen (*Musca domestica*), Küchenschaben (*Periplaneta americana*) und Heuschrecken (*Locusta* spp., *Schistocerca* spp.) stellen ein geeignetes biologisches Ausgangsmaterial für die Präparation biologischer Membranen dar, die Phthalsäurediamid-Rezeptoren enthalten.

Für das erfindungsgemäße Verfahren kann man beispielsweise präpariertes Nerven- oder Muskelgewebe von Insekten in einem biologischen Standardpuffer bei neutralem pH homogenisieren. Membranen, die Phthalsäurediamid-Rezeptoren enthalten, können durch differenzielle Zentrifugation gewonnen werden. In einem ersten Zentrifugationsschritt bei etwa 800 x g werden intakte Zellen, große Zellmembranfragmente und Zellkerne abgetrennt. Bei etwa 8000 x g werden Mitochondrien und ein großer Teil der Zellmembranen aus dem Homogenat entfernt. Membranen, die Phthalsäurediamid-Rezeptoren enthalten, werden bei 10⁵ x g sedimentiert, in einem geeigneten biologischen Puffer suspendiert und bis zu ihrer Verwendung bei -20 bis -80°C gelagert.

Bevorzugt werden für das erfindungsgemäße Verfahren Membranpräparationen verwendet, die ausgehend von adulten Organismen der Spezies *Heliothis virescens* oder deren L5-Larven bzw. der Spezies *Musca domestica* hergestellt wurden.

In einer weiteren Ausführungsform des erfindungsgemäßen Verfahrens können Membranen von Wirtszellen, insbesondere CHO-Zellen, die mit einer cDNA, basierend auf dem Gen CG10844 aus *Drosophila melanogaster* aus der Literaturstelle [Xu, X., et al.; Biophys.J. 78, 1270-1281 (2000)] oder auf dem entsprechenden Gen anderer Organismen, transient oder stabil transfiziert sind, eingesetzt werden. Es liegt im fachmännischen Können, die betreffende cDNA bereitzustellen und in geeigneten Wirtszellen zu exprimieren. Das entsprechende Genprodukt stellt das molekulare Target der Verbindungen gemäß der allgemeinen Formel (I) dar. Als Wirtszellen werden vorzugsweise Zellen verwendet, die das molekulare Target der Phthalsäurediamide nicht exprimieren, wie beispielweise CHO, HEK-293, 3T3, SF9 oder Schneider 2 Zellen.

### Beispiele

### Beispiel 1

### Herstellung einer markierten Verbindung gemäß der allgemeinen Formel (I) durch Austausch von Wasserstoff gegen Tritium

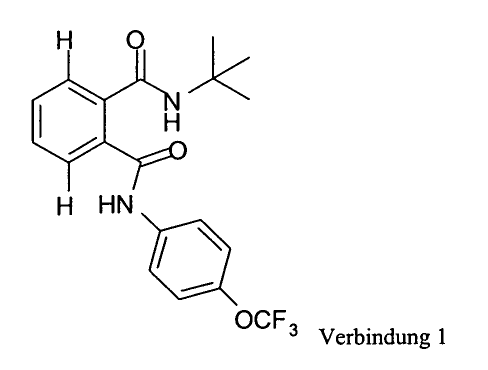

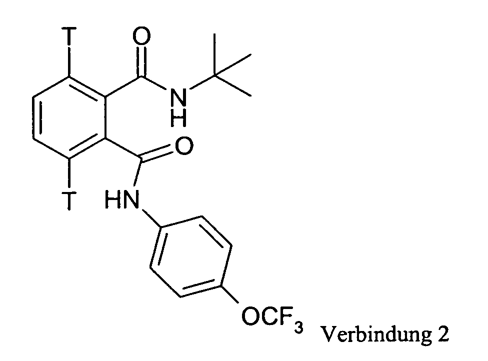

Die Markierung der in diesem Beispiel dargestellten Verbindung erfolgte nach Standardverfahren aus der Literatur. Nach diesem Verfahren wurde die nicht markierte Ausgangsverbindung (Verbindung 1) mit Hilfe des Katalysators (1,5-Cyclooctadien)(pyridin)(tricyclohexylphosphin)-iridium(I) hexafluor-phosphat tritiiert. Das Edukt (Verbindung 1) wurde in Dichlormethan gelöst und unter ständigem Rühren in Anwesenheit einer Tritiumatmosphäre tritiiert. Anschließend erfolgte die Abtrennung des Katalysators durch HPLC. Hierdurch erhielt man die Verbindung 2, welche als markierter Ligand in die Bindungsexperimente gemäß Beispiel 2 eingesetzt wurde.

Die insektizide Aktivität der tritiierten Verbindung 2 wurde in dem nachstehend beschriebenen biologischen Test überprüft. Sie unterschied sich in ihrer insektiziden Wirkung nicht von der unmarkierten Ausgangsverbindung (Verbindung 1).

### Spodoptera frugiperda-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind. Nach der gewünschten Zeit wird die Abtötung in % bestimmt.

### Beispiel 2

### Bindungstest an Membranpräparationen aus Insekten zum Identifizieren pestizider Wirkstoffe

Im Folgenden wird ein Verfahren zur Bestimmung der Wechselwirkung von Prüfsubstanzen mit der Bindungsstelle der Verbindungen der allgemeinen Formel (I) in Insektenmembranen beschrieben. Das Radioliganden-Bindungsverfahren ist automatisierbar und geeignet zum Substanz-Screening im Hochdurchsatz-Verfahren.
1. Präparation mikrosomaler Membranen:
   1.1 Biologisches Material:
      1.1.1 *Heliothis virescens* L5 Larven, adulte *Heliothis virescens,* adulte *Musca domestica.*
      1.1.2 CHO-Zellen, die mit einer cDNA des Gens CG8272-RA aus *Drosophila melanogaster* transient oder stabil transfiziert sind.
   1.2 Puffer und Reagenzien:
      Puffer 1: 25 mM K-PIPES, pH 7,4, 0,3 M Saccharose, 0,2 M KCl, 0,1 mM EGTA, Aliquot eines kommerziellen Proteaseinhibitor-Cocktails.
      Puffer 2: 20 mM Tris/HCl, pH 7,4, 0,3 M Saccharose, 1,0 mM Dithiothreitol, 0,8 % (w/v) Rinderserumalbumin.
   1.3 Präparation:
      1.3.1 Insektengewebe:
         Larven bzw. die isolierten Thoraces adulter Insekten wurden in Puffer 1 (10 ml g⁻¹ biologisches Material) mittels eines Ultra-Turrax homogenisiert. Das Homogenat wurde durch 2 Lagen Miracloth-Gaze filtriert und für 10 min bei 500 x g und 4°C zentrifugiert. Der Überstand wurde erneut durch 4 Lagen Miracloth-Gaze filtriert und anschließend für 20 min bei 8000 x g und 4°C zentrifugiert. Der Überstand wurde schließlich für 60 min bei 100000 x g und 4°C ultrazentrifugiert, um die mikrosomalen Membranen zu sedimentieren.
         Das Sediment wurde in einem kleinen Volumen Puffer 2 mittels eines Potter-Homogenisators resuspendiert und der Proteingehalt durch Verdünnung auf 10 - 20 mg ml⁻¹ eingestellt. Aliquots wurden in flüssigem Stickstoff eingefroren und bei -80°C gelagert.
      1.3.2 Membranen transfizierter Zellen:
         Transfizierte CHO-Zellen wurden in Puffer 2 suspendiert und lysiert. Das Lysat wurde zunächst 5 min bei 500 x g und 4°C zentrifugiert. Der zellfreie Überstand wurde 60 min bei 100000 x g und 4°C zentrifugiert, um die Zellmembranen zu sedimentieren.
         Das Sediment wurde in einem kleinen Volumen Puffer 2 mittels eines Potter-Homogenisators resuspendiert und der Proteingehalt durch Verdünnung auf 5 - 10 mg ml⁻¹ eingestellt. Aliquots wurden in flüssigem Stickstoff eingefroren und bei -80°C gelagert.
2. Bindungstest:
   2.1 Puffer und Reagenzien:
      Puffer 3: 10 mM HEPES, pH 7,4, 800 µM CaCl₂ x 2 H₂O, 10 mM ATP, 1,5 M KCl, 0,05 % Rinderserumalbumin.
      Puffer 4: 10 mM HEPES, pH 7,4, 150 mM KCl, vorgekühlt auf 4°C.
   2.2 Testdurchführung:
      Mikrotiterplatten (96 Kavitäten) wurden mit je 50 µl/Kavität von Testsubstanzlösungen geeigneter Konzentration (in Wasser/1 % DMSO Endkonzentration, Negativkontrollen 1 % DMSO, gegebenenfalls Referenzverbindungen als Positivkontrollen) befüllt.
      Je 100 µl Proteinlösung (verdünnt in Puffer 3, Endkonzentration: 50 µg Protein/Testansatz) und 100 µl Radioligand-Lösung (verdünnt in Puffer 3, Endkonzentration im Bereich der Dissoziationskonstante des Liganden: typischerweise 2 - 5 nM) wurden zugegeben.
      Die Testplatten wurden für genau 2 h bei 22°C inkubiert. Anschließend wurden die Testansätze über GF/B-Filter (mit 0,1 % Polyethylenimin befeuchtet) filtriert und die Filter mit 2,0 ml Puffer 4 gewaschen. Die Filter wurden mit Szintillatorlösung versetzt und die gebundene Radioaktivität im Flüssigszintillationszähler gemessen.

Die spezifische Bindung ergibt sich als Differenz aus der gemessenen Gesamtradioaktivität der Testansätze (Bₜₒₜₐₗ) und der gebundenen Radioaktivität von Kontrollansätzen, in denen der Radioligand durch einen mindestens 10⁴-fachen Überschuss eines geeigneten unmarkierten Liganden von seinen spezifischen Bindungsstellen quantitativ verdrängt wurde (= B_{unspezifisch}). Der Anteil der unspezifischen Bindung an der Gesamtbindung liegt typischerweise bei 5 bis 25 %, die maximale spezifische Bindung beträgt zwischen 1 und 3 pmol mg⁻¹ des eingesetzen Membranproteins.

Eine konzentrationsabhängige Verminderung der spezifischen Radioligandenbindung dient zur Identifizierung aktiver Testsubstanzen.

Bei Verwendung des Verfahrens im Hochdurchsatz-Screening chemischer Substanzbibliotheken werden die Testverbindungen einzeln oder als Mischung von 8 - 12 Einzelverbindungen in einer festgelegten Konzentration, zum Beispiel 10 µM, getestet. Die signifikante Reduktion der Bindung des Radioliganden im Vergleich zu Negativkontrollen (> 30 %) dient zur Identifizierung aktiver Testverbindungen, die mit den Ligandenbindestellen spezifisch wechselwirken.

### Beispiel 3

### Konzentrationsabhängige Inhibition der Bindung einer markierten Verbindung der allgemeinen Formel (I) (Verbindung 2) durch eine Testverbindung (Verbindung 1)

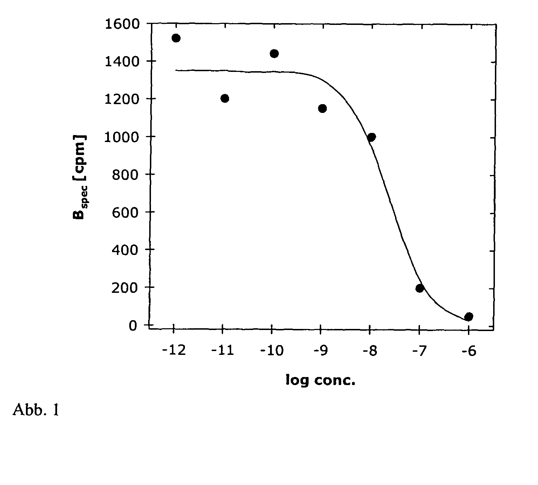

Die Abbildung 1 stellt die Inhibition der spezifischen Bindung der Verbindung 1 durch die Testsubstanz (Verbindung 2) als Funktion der Konzentration dar.

Der Wendepunkt der an die Datenpunkte angepassten sigmoiden Kurve (IC₅₀) beschreibt die Substanzkonzentration, bei der 50 % der spezifischen Ligandenbindung inhibiert werden: IC₅₀ = 25.1 nM (r² = 0.957).

## Patentansprüche

1. Verfahren zum Identifizieren pestizider Wirkstoffe, **dadurch gekennzeichnet, dass** man die Verdrängung einer i) markierten Verbindung gemäß der nachstehenden allgemeinen Formel (I) oder ii) einer markierten Verbindung, die an die gleiche Bindungsstelle bindet wie eine Verbindung gemäß der allgemeinen Formel (I) an Membranpräparationen oder Zelllinien, die das molekulare Target der Verbindungen gemäß der allgemeinen Formel (I) enthalten, misst: 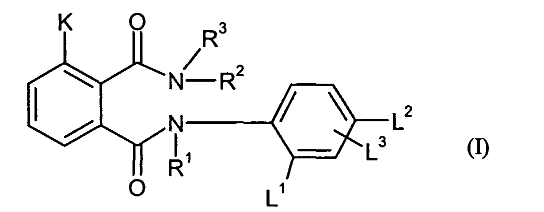 in welcher
K für Halogen, Cyano, Alkyl, Halogenalkyl, Alkoxy oder Halogenalkoxy steht,
R¹, R², R³ jeweils unabhängig voneinander für Wasserstoff, Cyano, für gegebenenfalls durch Halogen substituiertes C₃-C₈-Cycloalkyl oder für eine Gruppe der Formel M¹-Qₖ stehen, in welcher
M¹ für gegebenenfalls substituiertes Alkylen, Alkenylen oder Alkinylen steht,
Q für Wasserstoff, Halogen, Cyano, Nitro, Halogenalkyl, jeweils gegebenenfalls substituiertes C₃-C₈-Cycloalkyl, Alkylcarbonyl oder Alkoxycarbonyl, jeweils gegebenenfalls substituiertes Phenyl, Hetaryl oder für eine Gruppe T-R⁴ steht, in welcher
T für -O-, -S(O)ₘ- oder 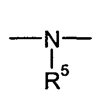 steht,
R⁴ für Wasserstoff, jeweils gegebenenfalls subsitutiertes Alkyl, Alkenyl, Alkinyl, Cycloalkyl, Cycloalkyl-alkyl, Alkoxyalkyl, Alkylcarbonyl, Alkoxycarbonyl, Phenyl, Phenylalkyl, Phenylalkoxy, Hetaryl, Hetarylalkyl steht,
R⁵ für Wasserstoff, für jeweils gegebenenfalls substituiertes Alkylcarbonyl, Alkoxycarbonyl, Phenylcarbonyl oder Phenylalkoxycarbonyl steht,
k für die Zahlen 1 bis 4 steht,
m für die Zahlen 0 bis 2 steht,
oder
R¹ und R² gemeinsam einen gegebenenfalls substituierten vier- bis siebengliedrigen Ring bilden, der gegebenenfalls durch Heteroatome unterbrochen sein kann,
L¹ und L³ unabhängig voneinander für Wasserstoff, Halogen, Cyano oder jeweils gegebenenfalls substituiertes Alkyl, Alkoxy, Alk-S(O)ₘ₋ , Phenyl, Phenoxy oder Hetaryloxy stehen,
L² für Wasserstoff, Halogen, Cyano, jeweils gegebenenfalls substituiertes Alkyl, Alkenyl, Alkinyl, Halogenalkyl, Cycloalkyl, Phenyl, Hetaryl oder für die Gruppe M²-R⁶ steht, in welcher
_{M}2 für -O- oder -S(O)ₘ- steht,
R⁶ für jeweils gegebenenfalls substituiertes Alkyl, Alkenyl, Alkinyl, Cycloalkyl, Phenyl oder Hetaryl steht,
oder
L¹ und L³ oder L¹ und L² gemeinsam einen gegebenenfalls substituierten fünf- bis sechsgliedrigen Ring bilden, der gegebenenfalls durch Heteroatome unterbrochen sein kann.

2. Verfahren gemäß Anspruch 1, **dadurch gekennzeichnet, dass**
K für Fluor, Chlor, Brom, Jod, Cyano, C₁-C₆-Alkyl, C₁-C₆-Halogenalkyl, C₁-C₆-Alkoxy oder C₁-C₆-Halogenalkoxy steht,
R¹, R², R³ jeweils unabhängig voneinander für Wasserstoff, Cyano, für gegebenenfalls durch Halogen substituiertes C₃-C₆-Cycloalkyl oder für eine Gruppe der Formel M ¹-Qₖ stehen, in welcher
M¹ für C₁-C₈-Alkylen, C₃-C₆-Alkenylen oder C₃-C₆-Alkinylen steht,
Q für Wasserstoff, Halogen, Cyano, Nitro, Halogenalkyl oder für gegebenenfalls durch Fluor, Chlor, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen substituiertes C₁-C₆-Alkylcarbonyl oder C₁-C₆-Alkoxycarbonyl oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl oder Hetaryl mit 5 bis 6 Ringatomen oder für eine Gruppe T-R⁴ steht, in welcher
T für -O-, -S(O)ₘ- oder 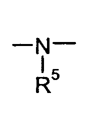 steht,
R⁴ für Wasserstoff, für jeweils gegebenenfalls durch Fluor und/oder Chlor substituiertes C₁-C₈-Alkyl, C₃-C₈-Alkenyl, C₃-C₈-Alkinyl, C₃-C₈-Cycloalkyl, C₃-C₈-Cycloalkyl-C₁-C₂-alkyl, C₁-C₆-Alkylcarbonyl, C₁-C₆-Alkoxycarbonyl, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, C₁-C₄-Phenylalkyl, C₁-C₄-Phenylalkyloxy, Hetaryl oder Hetarylalkyl, wobei Hetaryl mit 5 bis 6 Ringatomen steht,
R⁵ für Wasserstoff, für jeweils gegebenenfalls durch Fluor und/oder Chlor substituiertes C₁-C₆-Alkylcarbonyl, C₁-C₆-Alkoxycarbonyl, für jeweils gegebenenfalls einfach bis vierfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenylcarbonyl oder Phenyl-C₁-C₄-alkyloxycarbonyl steht,
k für die Zahlen 1 bis 3 steht,
m für die Zahlen 0 bis 2 steht,
oder
R¹ und R² einen fünf- bis sechsgliedrigen Ring bilden, der gegebenenfalls durch ein Sauerstoff- oder Schwefelatom unterbrochen sein kann,
L¹ und L³ unabhängig voneinander für Wasserstoff, Fluor, Chlor, Brom, Jod, Cyano, C₁-C₆-Alkyl, C₁-C₄-Halogenalkyl, C₁-C₆-Alkoxy, C₁-C₄-Halogenalkoxy, C₁-C₄-Alkyl-S(O)ₘ₋ , C₁-C₄-Haloalkyl-S(O)ₘ₋ , für jeweils gegebenenfalls einfach bis dreifach durch Fluor, Chlor, Brom, C₁-C₆-Alkyl, C₁-C₆-Alkoxy, C₁-C₄-Halogenalkyl, C₁-C₄-Halogenalkoxy, Cyano oder Nitro substituiertes Phenyl, Phenoxy, Pyridyloxy, Thiazolyloxy oder Pyrimidyloxy stehen,
L² für Wasserstoff, Fluor, Chlor, Brom, Jod, Cyano, für jeweils gegebenenfalls durch Fluor und/oder Chlor substituiertes C₁-C₁₀-Alkyl, C₂-C₁₀-Alkenyl, C₂-C₆-Alkinyl, für gegebenenfalls durch Fluor oder Chlor substituiertes C₃-C₆-Cycloalkyl, für jeweils gegebenenfalls einfach bis dreifach durch Fluor, Chlor, Brom, C₁-C₆-Alkyl, C₁-C₆-Alkoxy, C₁-C₄-Halogenalkyl, C₁-C₄-Halogenalkoxy, Cyano oder Nitro substituiertes Phenyl, Pyridyl, Thienyl, Pyrimidyl oder Thiazolyl, oder für eine Gruppe M²-R⁶ steht, in welcher
M² für -O- oder -S(O)ₘ- steht,
R⁶ für jeweils gegebenenfalls durch Fluor und/oder Chlor substituiertes C₁-C₈-Alkyl, C₂-C₈-Alkenyl, C₃-C₆-Alkinyl oder C₃-C₆-Cycloalkyl, für jeweils gegebenenfalls einfach bis dreifach durch Fluor, Chlor, Brom, C₁-C₆-Alkyl, C₁-C₆-Alkoxy, C₁-C₄-Halogenalkyl, C₁-C₄-Halogenalkoxy, Cyano oder Nitro substituiertes Phenyl, Pyridyl, Pyrimidyl oder Thiazolyl steht,
oder
L¹ und L³ oder L² und L³ gemeinsam jeweils einen gegebenenfalls durch Fluor und/oder C₁-C₂-Alkyl substituierten fünf- bis sechsgliedrigen Ring bilden, der gegebenenfalls durch ein oder zwei Sauerstoffatome unterbrochen sein kann.

3. Verfahren gemäß Anspruch 1, **dadurch gekennzeichnet, dass**
K für Chlor, Brom oder Jod steht,
R¹, R², R³ jeweils unabhängig voneinander für Wasserstoff oder für eine Gruppe der Formel M¹-Qₖ stehen, in welcher
M¹ für C₁-C₈-Alkylen, C₃-C₆-Alkenylen oder C₃-C₆-Alkinylen steht,
Q für Wasserstoff, Fluor, Chlor, Cyano, Trifluormethyl, C₃-C₆-Cyeloalkyl oder für eine Gruppe T-R⁴ steht, in welcher
T für -O- oder -S(O)ₘ- steht,
_{R}4 für Wasserstoff, jeweils gegebenenfalls einfach bis dreifach durch Fluor und/oder Chlor substituiertes C₁-C₆-Alkyl, C₃-C₆-Alkenyl, C₃-C₆-Alkinyl oder C₃-C₆-Cycloalkyl steht,
k für die Zahlen 1 bis 3 steht,
m für die Zahlen 0 bis 2 steht,
L¹ und L³ unabhängig voneinander für Wasserstoff, Fluor, Chlor, Brom, Jod, Cyano, C₁-C₄-Alkyl, C₁-C₂-Halogenalkyl, C₁-C₄-Alkoxy, C₁-C₂-Halogenalkoxy, für jeweils gegebenenfalls einfach oder zweifach durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl oder Phenoxy stehen,
L² für Wasserstoff, Fluor, Chlor, Brom, Jod, Cyano, für jeweils gegebenenfalls einfach bis dreizehnfach durch Fluor und/oder Chlor substituiertes C₁-C₆-Alkyl, C₂-C₆-Alkenyl, C₂-C₆-Alkinyl, C₃-C₆-Cycloalkyl oder für eine Gruppe M²-R⁶ steht, in welcher
M² für -O- oder -S(O)ₘ- steht,
R⁶ für jeweils gegebenenfalls einfach bis dreizehnfach durch Fluor und/oder Chlor substituiertes C₁-C₆-Alkyl, C₂-C₆-Alkenyl, C₂-C₆-Alkinyl oder C₃-C₆-Cycloalkyl, für jeweils gegebenenfalls einfach bis zweifach durch Fluor, Chlor, Brom, C₁-C₄-Alkyl, C₁-C₄-Alkoxy, Trifluormethyl, Difluormethoxy, Trifluormethoxy, Cyano oder Nitro substituiertes Phenyl oder Pyridyl steht.

4. Verfahren gemäß Anspruch 1, **dadurch gekennzeichnet, dass** es sich bei Verbindung gemäß der allgemeinen Formel (I) um die folgende Verbindung handelt:

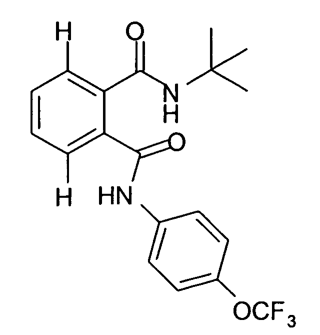

5. Verfahren gemäß einem der Ansprüche 1 bis 4, **dadurch gekennzeichnet, dass** die Verbindung gemäß der allgemeinen Formel (I) mit einem radioaktiven Isotop markiert ist.

6. Verfahren gemäß Anspruch 5, **dadurch gekennzeichnet, dass** die Verbindung gemäß der allgemeinen Formel (I) mit Tritium markiert ist.

7. Verfahren gemäß einem der Ansprüche 1 bis 6, **gekennzeichnet durch** die folgenden Schritte:
a1) Herstellen einer Membranpräparation ausgehend von adulten Organismen oder deren Larven, oder
a2) Herstellen einer Membranpräparation ausgehend von Zellen, die das molekulare Target der Verbindungen gemäß der allgemeinen Formel (I) exprimieren,
b) Herstellen einer Proteinlösung ausgehend von den besagten Membranpräparationen,
c) Inkontaktbringen der Proteinlösung mit einer markierten Verbindung gemäß der allgemeinen Formel (I) unter Bedingungen, die eine Bindung der markierten Verbindung mit dem molekularen Target erlauben,
d) Inkontaktbringen der im Schritt c) hergestellten Mischung mit einer unmarkierten Testsubstanz,
e) Messen, ob eine Verdrängung der markierten Verbindung **durch** die Testsubstanz stattgefunden hat,
und gegebenenfalls
f) Prüfen der Testsubstanz auf pestizide Eigenschaften.

8. Verfahren gemäß Anspruch 7, **dadurch gekennzeichnet, dass** im Schritt al) Membranpräparationen ausgehend von adulten Insekten der Spezies *Musca domestica* hergestellt werden.

9. Verfahren gemäß Anspruch 7, **dadurch gekennzeichnet, dass** im Schritt a1) Membranpräparationen ausgehend von adulten Insekten der Spezies *Heliothis virescens* oder deren Larven hergestellt werden.
